# EUROPÄISCHE PATENTSCHRIFT

(11) **EP 1 122 237 B1**
(45) Veröffentlichungstag und Bekanntmachung des Hinweises auf die Patenterteilung: **22.10.2003**
(21) Anmeldenummer: 01100087.4
(22) Anmeldetag: 11.01.2001
(51) Int. Cl.: C07C 69/34, C07C 67/39, C07C 67/08, C07C 69/75, C07C 69/675, D06M 13/224

(54) **Polycarbonsäuren, Verfahren zu deren Herstellung und deren Verwendung zur Behandlung von Cellulosefasern oder daraus hergestellten Textil- oder Papiermaterialien**
Polycarboxylic acids, process for their preparation and use for treating cellulose fibers and textile or paper materials produced therefrom
Acides polycarboxyliques,leur procédé de préparation et utilisation pour le traitement de fibres cellulosiques ou de matières textiles ou en papier obtenues à partir de ceux-ci

(30) Priorität: 24.01.2000 DE 10002877
(43) Veröffentlichungstag der Anmeldung: 08.08.2001
(73) Patentinhaber: Bayer Aktiengesellschaft, 51368 Leverkusen (DE)
(72) Erfinder: Gerle, Michael, Dr., 50127 Bergheim (DE); Ehlert, Hans-Albert, Singapore 259293 (SG); Franke, Günter, Dr., 42799 Leichlingen (DE); Meier, Helmut-Martin, Dr., 40883 Ratingen (DE)

(56) Entgegenhaltungen:
- EP-A- 0 688 897

## Beschreibung

Die Erfindung betrifft säurestabile Polycarbonsäuren, ein Verfahren zu deren Herstellung durch oxidative Spaltung von Doppelbindungen enthaltenden Verbindungen mit Wasserstoffperoxid oder Wasserstoffperoxid-abgebenden Verbindungen, die Verwendung der Polycarbonsäuren zur Behandlung von Cellulosefasern oder daraus hergestellten Textil- oder Papiermaterialien sowie die derart behandelten Cellulosefasern, Textil- oder Papiermaterialien.

Textilien aus Cellulosefasern wie Baumwolle besitzen gegenüber synthetischen Fasern den Vorteil, hydrophil zu sein, was sich in hoher Feuchtigkeitsaufnahmefähigkeit und gutem Tragekomfort äußert. Ursache für die hohe Feuchtigkeitsaufnahmefähigkeit sind die quellbaren amorphen Bereiche der Cellulosefasern. Nachteilig ist allerdings die Tatsache, dass durch Waschen oder Schweiß gequollene Cellulose knittert und durch thermische und mechanische Behandlung wieder geglättet werden muss. Außerdem schrumpft Baumwolle beim Waschen, wodurch Textilien ihre ursprüngliche Form verlieren. Um diese Nachteile zu beseitigen, werden cellulosehaltige Fasern seit vielen Jahren mit Produkten behandelt, die durch Reaktion mit den Hydroxylgruppen der Cellulose die amorphen Teile der Fasern zum Teil vernetzen. Durch diese Behandlung behält das Textil beim Tragen und Waschen seine Form. Bevorzugt werden als Vemetzer methylolisierte Harnstoff- oder Melaminderivate eingesetzt. Nachteilig bei diesen Verbindungen ist die Tatsache, dass sie bei der Ausrüstung und Benutzung des Textils Formaldehyd freisetzen können.

Es hat daher nicht an Versuchen gefehlt, alternative und ökologisch vorteilhaftere Produkte zur Behandlung von Textilien zu finden. Die Verwendung von Polycarbonsäuren wie Butantetracarbonsäure zur formaldehydfreien Textilausrüstung ist aus Text. Res. J. 37, 933 (1967) und US-A-4,820,307 prinzipiell bekannt.

Die Möglichkeit, Polycarbonsäuren durch oxidative Spaltung von Doppelbindungen enthaltenden Verbindungen herzustellen, ist ebenfalls bereits prinzipiell bekannt. So kann Butantetracarbonsäure durch oxidative Spaltung aus Tetrahydrophthalsäure mit Salpetersäure unter Vanadiumkatalyse hergestellt werden (J. Org. Chem. 30, 1488 (1965), DE-A-30 16 225, JP 59128350 A2). Nachteilig bei diesem Verfahren sind die aggressiven Reaktionsbedingungen, die dazu führen, dass ein Teil des eingesetzten Ausgangsmaterials sowie des Zwischen- und Endprodukts durch weitere Oxidationsreaktionen zersetzt werden. Außerdem entweichen bei der Reaktion giftige Stickoxide, die nur durch eine aufwendige Gaswäsche entfernt werden können. Als Nebenkomponenten werden bei dem Verfahren stickstoffhaltige Verbindungen gebildet, die bei der Verwendung als Textilvernetzer zu störenden Gelbfärbungen des ausgerüsteten Gewebes führen und nur durch aufwendiges Umkristallisieren entfernt werden können.

Besser geeignet als Oxidationsmittel ist daher Wasserstoffperoxid, da bei seiner Verwendung nur Wasser als Reaktionsprodukt entsteht. US-A-3,646,130 beschreibt die oxidative Spaltung von Cyclododecen mit Wasserstoffperoxid unter Verwendung von Re₂O₇ als Katalysator. Aus EP-A-0 122 804 ist ferner die oxidative Spaltung verschiedener Olefinen mit Wasserstoffperoxid unter Verwendung eines Katalysators bekannt, der aus H₂WO₄, H₃PO₄ und einem Phasentransferkatalysator hergestellt wird. In EP-A-0 123 495 werden verschiedene durch Dihydroxylierung von Olefinen erhältliche Zwischenprodukte unter Verwendung eines katalytischen Systems aus H₂WO₄ und H₃PO₄ mit Wasserstoffperoxid zu den entsprechenden Carbonsäuren gespalten.

EP-A-0 513 600 beschreibt die Herstellung von Carbonsäuren durch oxidative Aufarbeitung von mit Ozon umgesetzten Olefinen. Auch hier finden sich Hinweise zur Herstellung von Butantetracarbonsäure aus Tetrahydrophthalsäureanhydrid. Nachteilig bei diesem Verfahren ist aber die Verwendung von giftigem Ozon, dessen Herstellung außerdem energieaufwendig ist.

US-A-5,047,582 beschreibt die Herstellung von Polycarbonsäuren in einem zweistufigen Verfahren. In der ersten Stufe wird in einem nichtkatalysierten Verfahren aus einem Olefin die entsprechende Dihydroxylverbindung hergestellt, die in einer zweiten Stufe unter Verwendung verschiedener Übergangsmetallkatalysatoren in die entsprechende Polycarbonsäure gespalten wird.

EP-A-0 201 719 offenbart die Herstellung von Polycarbonsäuren wie Butantetracarbonsäure durch oxidative Spaltung von Olefinen wie Tetrahydrophthalsäureanhydrid mit Wasserstoffperoxid unter Verwendung von Wolfram- bzw. Molybdänsäure oder Wolfram- bzw. Molybdänheteropolysäuren.

EP-A-0 688 897 beschreibt die oxidative Spaltung von Tetrahydrophthalsäureanhydrid mit Wasserstoffperoxid unter Bildung von Butantetracarbonsäure sowie die Verwendung der so erhaltenen Butantetracarbonsäure zur Behandlung von Cellulosefasern.

In JP 08295649 A2 werden zur Herstellung von Butantetracarbonsäure Wolframverbindungen in Kombination mit stickstoffhaltigen heterocyclischen Carbonsäuren als Oxidationskatalysatoren verwendet.

Nachteilig an dem zuvor beschriebenen Stand der Technik ist die Tatsache, dass die erhaltene Butantetracarbonsäure keine ausreichende Säurestabilität in den anfallenden wässrigen Reaktionslösungen aufweist. Dies führt dazu, dass ein Teil der Butantetracarbonsäure als Feststoff ausfällt, der Rest aber in der wässrigen Phase gelöst bleibt. Derartige Lösungen mit Feststoffanteil sind aber für die Behandlung von Fasern oder Textilmaterialien ungeeignet. Die Butantetracarbonsäure muß daher durch Verdampfung des Wassers zunächst vollständig isoliert werden. Dies ist jedoch energieaufwendig und damit nicht ökonomisch. Zudem sind Vernetzer, die als Feststoff vorliegen, wenig attraktiv; die meisten gängigen Vernetzer für Cellulose werden aufgrund ihrer besseren Handhabbarkeit als wässrige Lösungen angeboten. Diese Nachteile sind der Grund dafür, dass Polycarbonsäuren wie Butantetracarbonsäure bisher keine kommerzielle Anwendung zur Ausrüstung von cellulosehaltigen Fasern oder daraus hergestellten Textilmaterialien gefunden haben.

Die Aufgabe der vorliegenden Erfindung bestand somit darin, ein Verfahren bereitzustellen, mit dem Polycarbonsäuren hergestellt werden können, die eine ausreichende Säurestabilität in wässriger Lösung besitzen und für die Vernetzung von Cellulosefasern geeignet sind.

Gegenstand der Erfindung ist ein Verfahren zur Herstellung von Polycarbonsäuren durch
1) Umsetzung von Verbindungen der Formeln (I) oder (II) 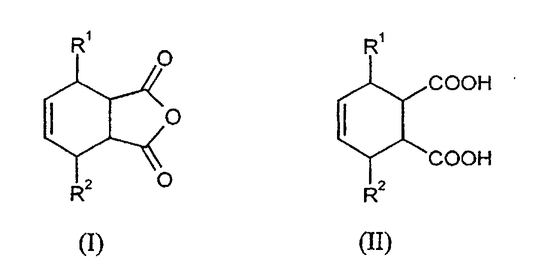 wobei
   - R¹ und R²: gleich oder verschieden sind und H oder geradkettiges oder verzweigtes C₁-C₅-Alkyl bedeuten,
   mit Verbindungen der Formel (III)

   R³XH (III)

   wobei
   - X: O, NH oder S und
   - R³: geradkettiges oder verzweigtes C₁-C₃₀-Alkyl,
   geradkettiges oder verzweigtes C₂-C₃₀-Alkenyl,
   C₅-C₁₂-Cycloalkyl,
   -CHR⁴COOH,
   worin
   R⁴ H, geradkettiges oder verzweigtes C₁-C₅-Alkyl, -CH₂OH, CH(OH)COOH oder -CH₂COOH bedeutet, oder
   -(CH₂CR⁵R⁶Y)ₙR⁷,
   wobei
   - Y: für O oder NR⁸ steht,
   - R⁵, R⁶, R⁷ und R⁸: unabhängig voneinander H, geradkettiges oder verzweigtes C₁-C₄-Alkyl, -CH₂OH oder -CH₂CH₂OH bedeuten und
   - n: eine ganze Zahl von 1 - 20 darstellt,
   bedeuten, und
2) anschließende Oxidation in Gegenwart von Wasserstoffperoxid oder einer Wasserstoffperoxid-freisetzenden Verbindung sowie eines Katalysators.

Gegenstand der Erfindung sind ferner die nach diesem Verfahren erhältlichen Polycarbonsäuren.

Als Edukte werden im erfindungsgemäßen Verfahren Verbindungen der Formeln (I) oder (II) eingesetzt. Hierbei handelt es sich um Tetrahydrophthalsäure, Tetrahydrophthalsäureanhydrid oder entsprechend substituierte Derivate hiervon. R¹ und R² sind gleich oder verschieden und stehen in den Formeln (I) und (II) bevorzugt für Wasserstoff oder Methyl. Besonders bevorzugt wird 1,2,3,6-Tetrahydrophthalsäureanhydrid zur Umsetzung mit Verbindung (III) eingesetzt.

Die im erfindungsgemäßen Verfahren eingesetzten Verbindungen der Formel (III) können, je nachdem welche der angegebenen Bedeutungen der Rest R³ annimmt, monofunktionell, bifunktionell, trifunktionell oder höherfunktionell sein.

Bevorzugt werden mindestens bifunktionelle Verbindungen der Formel (III) eingesetzt, bei denen
- X: O bedeutet und
- R³: -CHR⁴COOH,
worin
- R⁴: H, geradkettiges oder verzweigtes C₁-C₅-Alkyl, -CH₂OH, -CH(OH)COOH oder -CH₂COOH bedeutet, oder -(CH₂CR⁵R⁶Y)ₙR⁷,
wobei
- Y: für O oder NR⁸ steht und
- R⁵, R⁶, R⁷ und R⁸: unabhängig voneinander H, geradkettiges oder verzweigtes C₁-C₄-Alkyl, -CH₂OH oder -CH₂CH₂OH bedeuten und
- n: eine ganze Zahl von 1 bis 10, bevorzugt 1 bis 5 darstellt.

Besonders bevorzugt werden als bi- oder trifunktionelle Verbindungen der Formel (III) Ethylenglykol, Diethylenglykol, Triethylenglykol, Milchsäure, Glycerin, Trimethylolpropan oder 2,2-Bis(hydroxymethyl)propionsäure eingesetzt.

In einer bevorzugten Ausführungsform wird 1,2,3,6-Tetrahydrophthalsäureanhydrid mit einer Verbindung R³OH umgesetzt, wobei R³ für -(CH₂CR⁵R⁶Y)ₙR⁷ steht, wobei Y für O oder NR⁸ steht und R⁵, R⁶, R⁷ und R⁸ unabhängig voneinander H, geradkettiges oder verzweigtes C₁-C₄-Alkyl, -CH₂OH oder -CH₂CH₂OH bedeuten und n eine ganze Zahl von 1 bis 20, bevorzugt 1 bis 10 und insbesondere 1 bis 5 darstellt.

In einer besonders bevorzugten Ausführungsform wird 1,2,3,6-Tetrahydrophthalsäureanhydrid mit einer Verbindung R³OH umgesetzt, wobei R³ für (CH₂CH₂O)ₙH steht und n eine ganze Zahl von 1-5 und bevorzugt gleich 1, 2 oder 3 ist.

Die Umsetzung der Verbindungen (I) oder (II) mit der Verbindung (III) in Schritt 1) des erfindungsgemäßen Verfahrens erfolgt bei einer Temperatur von 50 bis 250°C, bevorzugt 90 bis 150°C, und einem Druck von 0 bis 10 bar, bevorzugt 0,5 bis 2 bar. Die Reaktionszeit liegt üblicherweise bei 0,5-24 Stunden, bevorzugt bei 1 bis 3 Stunden. Die Umsetzung wird vorzugsweise in Gefäßen und Reaktoren mit Rührvorrichtung durchgeführt. Die Verbindungen (I) bzw. (II) und (III) können dabei in beliebiger Reihenfolge zugegeben werden, bevorzugt werden sie nicht in Lösung eingesetzt sondern als Feststoffe oder Flüssigkeiten. Die Umsetzung kann in Gegenwart, aber auch in Abwesenheit eines Katalysators durchgeführt werden. Sofern ein Katalysator verwendet wird, können übliche Veresterungskatalysatoren, wie Säuren oder Basen, oder Metallkatalysatoren, wie z.B. Titan- oder Zinn-Verbindungen, eingesetzt werden.

Das nach der Umsetzung in Schritt 1) erhaltene Reaktionsgemisch wird anschließend in Schritt 2) direkt einer oxidativen Spaltung unter Einsatz von Wasserstoffperoxid oder einer in-situ Wasserstoffperoxid-freisetzenden Verbindung als Oxidationsmittel unterzogen. Eine Aufarbeitung des Reaktionsgemisches vor der oxidativen Spaltung ist vorteilhafterweise nicht nötig, kann aber durchgeführt werden.

Die Oxidation in Schritt 2) des erfindungsgemäßen Verfahrens stellt eine oxidative Spaltung der in den Reaktionsprodukten von Schritt 1) enthaltenen Doppelbindungen dar unter Ausbildung zweier Carbonsäuregruppen an zwei benachbarten und dann durch eine Einfachbindung verknüpften Kohlenstoffatomen dar. Diese Oxidation wird in Gegenwart eines Katalysators durchgeführt. Hierbei handelt es sich bevorzugt um Wolfram- oder Molybdän-haltige Katalysatoren. Beispiele für geeignete Wolfram-haltige Katalysatoren sind Wolframoxid (WO₃), Wolframsäure (H₂WO₄ bzw. WO₃·H₂O), die Isopolysäuren und Heteropolysäuren des Wolframs, Alkalimetall-, Erdalkalimetall- oder Ammonium-Wolframate, bevorzugt Natriumwolframat (Na₂WO₄·2H₂O), Ammoniumparawolframat ((NH₄)₁₀W₁₂O₄₁·11H₂O) oder Ammoniummetawolframat ((NH₄)₆H₂W₁₂O₄₀). Beispiele für geeignete Molybdän-haltige Katalysatoren sind Molybdän(VI)-oxid, die Isopolysäuren und Heteropolysäuren des Molybdäns sowie Alkalimetall-, Erdalkalimetall- oder Ammonium-Molybdate.

Als Oxidationsmittel wird Wasserstoffperoxid oder eine in-situ Wasserstoffperoxid abgebende Verbindung eingesetzt. Als in-situ Wasserstoffperoxid abgebende Verbindungen können Wasserstoffperoxid-Additionsverbindungen wie Peroxid-Harnstoff-Addukte oder Perverbindungen wie Perborate, Percarbonate oder Persulfate in Form ihrer Alkalisalze eingesetzt werden, und zwar einzeln oder in Gemischen. Anwendung findet bevorzugt eine 30 bis 60 gew.-%ige Wasserstoffperoxid-Lösung. Besonders bevorzugt wird mit einer handelsüblichen 50 gew.-%igen Wasserstoffperoxid-Lösung gearbeitet.

Die oxidative Spaltung wird bei einer Temperatur von 50 bis 150°C, bevorzugt 70 bis 95°C und einem Druck von 0 bis 4 bar, bevorzugt 0,5 bis 2 bar durchgeführt. Die Reaktionszeit beträgt üblicherweise 0,5 bis 24 Stunden, bevorzugt 3 bis 10 Stunden. Die Reaktion wird üblicherweise in einem Gefäß oder Reaktor mit Rührvorrichtung durchgeführt. Üblicherweise wird das Wasserstoffperoxid oder die Wasserstoffperoxid-freisetzende Verbindung zu dem Reaktionsgemisch aus der ersten Stufe des erfindungsgemäßen Verfahrens einfach zugegeben.

Gegenstand der vorliegenden Erfindung sind die nach dem vorgenannten Verfahren erhältlichen Polycarbonsäuren. Sie fallen nach der oxidativen Spaltung wie beschrieben in wässriger Lösung an und können vorteilhafterweise ohne weitere Aufarbeitung zur Textilausrüstung, d.h. zur Hochveredelung der Cellulosefasern oder daraus hergestellten Textilmaterialien eingesetzt werden. Es ist jedoch auch möglich, die Polycarbonsäuren durch Verdampfen des Wassers oder durch Fällung zu isolieren.

Der Vorteil der so erhaltenen Polycarbonsäuren besteht darin, dass sie gut wasserlöslich und säurestabil sind, d h. auch bei niedrigen Temperaturen sind sie im Gegensatz zu Butantetracarbonsäure gegen Ausfällungen stabil. Der pH-Wert der nach Schritt 2) des erfindungsgemäßen Verfahrens erhaltenen Polycarbonsäurelösung liegt im Bereich von 0 bis 2, bevorzugt ist der pH-Wert kleiner oder gleich 1. Je nach Verwendungszweck kann der pH-Wert dieser Polycarbonsäurelösungen noch mit basisch reagierenden Verbindungen auf einen pH-Wert von bis zu 10, bevorzugt auf einen pH-Wert im Bereich von 3 bis 5 eingestellt werden.

Je nachdem, ob die im erfindungsgemäßen Verfahren eingesetzte Verbindung der Formel R³XH mono-, bi-, tri- oder höherfunktionell ist und je nachdem, in welchem molaren Verhältnis die Verbindungen der Formeln (I) oder (II)_zu den Verbindungen der Formel (III) eingesetzt werden, können bei der Umsetzung Polycarbonsäuregemische entstehen.

Bei Einsatz einer monofunktionellen Verbindung R³XH entstehen über die Zwischenstufe der Formel (IV) 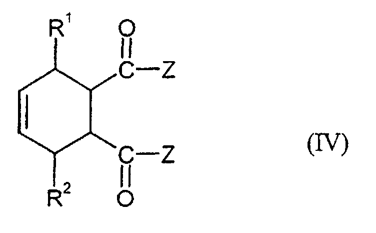 wobei entweder ein Rest Z für OH und ein Rest Z für XR³ stehen oder beide Reste für XR³ stehen, die Di- oder Tricarbonsäuren der Formel (V).

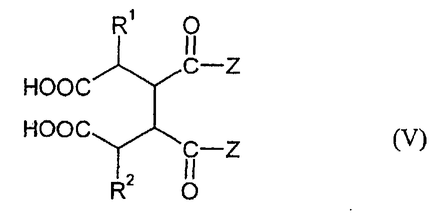

Im Fall bifunktioneller Verbindungen R³XH mit X gleich O, NH oder S ergibt sich bereits eine deutlich größere Zahl möglicher Polycarbonsäuren. Für den Fall, dass R³ beispielsweise für einen Rest (CH₂CR⁵R⁶Y)ₙH darstellt, wobei Y für O oder NR⁸ steht und R⁵, R⁶ und R⁸ unabhängig voneinander H oder geradkettiges oder verzweigtes C₁-C₄-Alkyl bedeuten und n eine ganze Zahl von 1 bis 20, bevorzugt 1 bis 10, besonders bevorzugt 1 bis 5 darstellt, und die Verbindung der Formel (I) oder (II) in einem molaren Verhältnis von 2:1 zur Verbindung (III) eingesetzt wird, entsteht über die Zwischenstufe der Formel (VI) 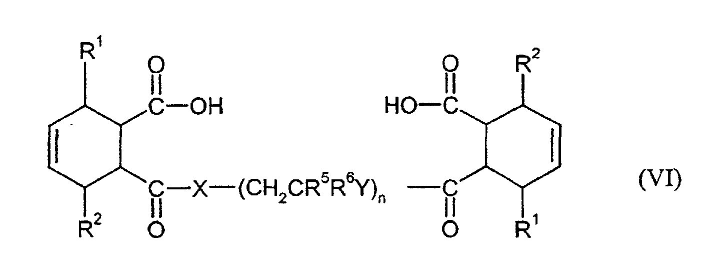 wobei
- X: für O, NH oder S steht,
- Y: für O oder NR⁸ steht,
- R⁵, R⁶ und R⁸: unabhängig voneinander H oder geradkettiges oder verzweigtes C₁-C₄-Alkyl bedeuten und
- n: eine ganze Zahl von 1 bis 20, bevorzugt 1 bis 10 und besonders bevorzugt von 1 bis 5 darstellt, als Hauptprodukt eine Hexacarbonsäure der Formel (VII),
wobei
- X, R⁵, R⁶ und Y: die für diese Variante bereits genannten Bedeutungen besitzen.

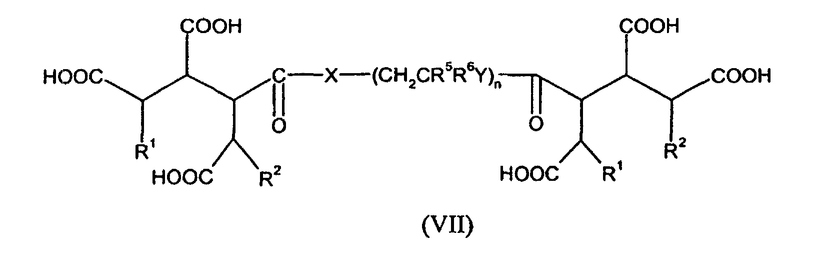

Gegenstand der Erfindung ist ferner die Verwendung der nach dem erfindungsgemäßen Verfahren erhältlichen Polycarbonsäuren zur Ausrüstung von cellulosehaltigen Fasern oder daraus hergestellten Textil- oder Papiermaterialien. Hierbei werden die erfindungsgemäßen Polycarbonsäuren bevorzugt direkt in Form der wässrigen Lösungen eingesetzt, die nach dem erfindungsgemäßen Verfahren anfallen.

Gegenstand der Erfindung ist ferner ein Verfahren zur Ausrüstung von cellulosehaltigen Fasern oder daraus hergestellten Textil- oder Papiermaterialien, dadurch gekennzeichnet, dass die cellulosehaltigen Fasern oder daraus hergestellten Textil- oder Papiermaterialien mit einer wässrigen Flotte behandelt werden, die die erfindungsgemäßen Polycarbonsäuren sowie gegebenenfalls Katalysatoren und Textilhilfsmittel, bevorzugt Weichmacher, Appreturmittel, Hydrophobiermittel, Oleophobiermittel, Flammschutzmittel oder pH-Regulatoren enthält.

Durch Reaktion mit den Hydroxylgruppen der Cellulose vernetzen die erfindungsgemäßen Polycarbonsäuren die amorphen Teile der Fasern teilweise. Durch diese Knitter- und Bügelfreiausrüstung wird sichergestellt, dass das Textil beim Tragen und Waschen seine Form behält.

Bei der Ausrüstung der cellulosehaltigen Fasern oder der daraus hergestellten Textil- oder Papiermaterialien können als Katalysatoren beispielsweise Alkalimetallhypophosphite und Alkalimetallphosphite, bevorzugt Natrium-hypophosphit oder Natriumphosphit, eingesetzt werden. Derartige Alkalimetallhypophosphite und Alkalimetallphosphite sind in der US-A-4,820,307 beschrieben. Ferner kann als Katalysator auch Cyanamid oder eine Verbindung der Formel (VIII) 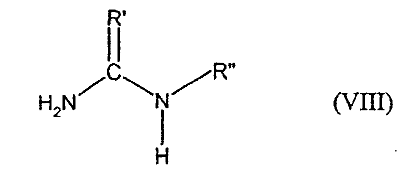 wobei
- R': für NH, O oder S steht
und
- R": CN oder H bedeutet,
verwendet werden. Cyanamid und die Verbindungen der Formel (VIII) sind in der US-A-5,205,836 beschrieben.

Die Behandlung der cellulosehaltigen Fasern oder der daraus hergestellten Textiloder Papiermaterialien mit der Flotte kann nach dem Fachmann geläufigen Methoden wie Fouladieren, Sprühen, Pflatschen oder Schäumen erfolgen. Üblicherweise wird mit einer Flottenkonzentration von 10 bis 100 g Wirkstoff pro Liter Flotte, bevorzugt 30 bis 70 g Wirkstoff pro Liter Flotte gearbeitet.

Nach erfolgter Ausrüstung werden die cellulosehaltigen Fasern oder daraus hergestellten Textil- oder Papiermaterialien bei Temperaturen von 100 bis 130°C getrocknet. Bevorzugt wird diese Trocknung bis zur Erreichung eines Restfeuchtegehalts von 1 bis 10 Gew.-%, bevorzugt 2 bis 6 Gew.-% und insbesondere 3 bis 4 Gew.-% durchgeführt. Anschließend wird bei Temperaturen von 130 bis 220°C, bevorzugt 140 bis 180°C eine Wärmebehandlung durchgeführt. Die Schritte der Trockung und Wärmebehandlung können auch in einer Stufe durchgeführt werden.

Mit dem erfindungsgemäßen Verfahren können jegliche cellulosehaltigen Fasern oder daraus hergestellte Textil- oder Papiermaterialien behandelt werden. Bei den cellulosehaltigen Fasern kann es sich beispielsweise um Textilfasern aus natürlicher oder regenerierter Cellulose oder aus Celluloseacetat handelt. Das Verfahren kann auch zur Ausrüstung von Mischungen aus cellulosehaltigen und synthetischen Fasern eingesetzt werden. Behandelt werden beispielsweise Baumwolle, Leinen, Kunstseiden oder Papier.

Gegenstand der Erfindung sind somit auch die cellulosehaltigen Fasern oder die daraus hergestellten Textil- oder Papiermaterialien, die mit den erfindungsgemäßen Polycarbonsäuren behandelt wurden.

### Beispiele:

### I Herstellung der Polycarbonsäuren

### Vergleichsbeispiel 1 nach EP-A-0 201 719, Example 1:

In einem Rührgefäß werden 30,4 g (0,20 mol) Tetrahydrophthalsäureanhydrid und 60 g Wasser auf 100°C erhitzt und eine halbe Stunde bei dieser Temperatur verrührt. Die Lösung wird auf 70°C abgekühlt, 1,0 g (4,0 mmol) Wolframsäure zugesetzt, tropfenweise 15 g (0,265 mol) 60%iges Wasserstoffperoxid zudosiert und 2 h bei 70°C gehalten . Anschließend werden 42 g (0,741 mol) 60 %iges Wasserstoffperoxid hinzugegeben, auf 90°C erhitzt und 10 h bei dieser Temperatur gehalten. Nach dem Abkühlen der Reaktionslösung auf Raumtemperatur fällt ein Teil der gebildeten Carbonsäure aus, während der Rest im Wasser gelöst bleibt.

### Beispiel A1:

In einem Rührgefäß werden 228,2 g (1,5 mol) Tetrahydrophthalsäureanhydrid und 46,6 g (0,75 mol) Ethylenglykol unter Rühren auf 100°C erhitzt. Durch die freiwerdende Reaktionswärme steigt die Temperatur der Reaktionsmischung auf 130°C. Nach dem Abkühlen auf 90°C werden 450 g Wasser hinzugefügt und anschließend noch 7,5 g (30 mmol) Wolframsäure zugesetzt. Über einen Zeitraum von 6 h werden insgesamt 510,3 g (7,5 mol) 50 %iges Wasserstoffperoxid zudosiert. Anschließend wird 10 h bei dieser Temperatur nachgerührt. Nach dem Abkühlen erhält man eine klare gelbliche Polycarbonsäurelösung. Vor der Verwendung zur Ausrüstung der Cellulose-Fasern werden noch 53,0 g (0,66 mol) 50 %ige Natronlauge hinzugefügt.

### Beispiel A2:

In einem Rührgefäß werden 228,2 g (1,5 mol) Tetrahydrophthalsäureanhydrid und 69,8 g (1,125 mol) Ethylenglykol unter Rühren auf 100°C erhitzt. Durch die freiwerdende Reaktionswärme steigt die Temperatur der Reaktionsmischung auf 130°C.

Nach dem Abkühlen auf 90°C werden 450 g Wasser hinzugefügt und anschließend noch 7,5 g (30 mmol) Wolframsäure zugesetzt. Über einen Zeitraum von 6 h werden insgesamt 510,3 g (7,5 mol) 50 %iges Wasserstoffperoxid zudosiert. Anschließend wird 10 h bei dieser Temperatur nachgerührt. Nach dem Abkühlen erhält man eine klare gelbliche Polycarbonsäurelösung. Vor der Verwendung zur Ausrüstung der Cellulose-Fasern werden noch 49,5 g (0,62 mol) 50 %ige Natronlauge hinzugefügt.

### Beispiel A3:

In einem Rührgefäß werden 228,2 g (1,5 mol) Tetrahydrophthalsäureanhydrid und 46,0 g (0,5 mol) Glycerin unter Rühren auf 100°C erhitzt. Durch die freiwerdende Reaktionswärme steigt die Temperatur der Reaktionsmischung auf 120°C. Nach dem Abkühlen auf 90°C werden 450 g Wasser hinzugefügt und anschließend noch 7,5 g (30 mmol) Wolframsäure zugesetzt. Über einen Zeitraum von 6 h werden insgesamt 510,3 g (7,5 mol) 50 %iges Wasserstoffperoxid zudosiert. Anschließend wird 10 h bei dieser Temperatur nachgerührt. Nach dem Abkühlen erhält man eine klare gelbliche Polycarbonsäurelösung. Vor der Verwendung zur Ausrüstung der Cellulose-Fasern werden noch 54,4 g (0,68 mol) 50 %ige Natronlauge hinzugefügt.

### Beispiel A4:

In einem Rührgefäß werden 228,2 g (1,5 mol) Tetrahydrophthalsäureanhydrid und 67,1 g (0,5 mol) Trimethylolpropan unter Rühren auf 100°C erhitzt. Durch die freiwerdende Reaktionswärme steigt die Temperatur der Reaktionsmischung auf 120°C. Nach dem Abkühlen auf 90°C werden 450 g Wasser hinzugefügt und anschließend noch 7,5 g (30 mmol) Wolframsäure zugesetzt. Über einen Zeitraum von 6 h werden insgesamt 510,3 g (7,5 mol) 50 %iges Wasserstoffperoxid zudosiert. Anschließend wird 10 h bei dieser Temperatur nachgerührt. Nach dem Abkühlen erhält man eine klare gelbliche Polycarbonsäurelösung. Vor der Verwendung zur Ausrüstung der Cellulose-Fasern werden noch 46,0 g (0,57 mol) 50 %ige Natronlauge hinzugefügt.

### Beispiel A5:

In einem Rührgefäß werden 228,2 g (1,5 mol) Tetrahydrophthalsäureanhydrid und 46,6 g (0,75 mol) Ethylenglykol unter Rühren auf 100°C erhitzt. Durch die freiwerdende Reaktionswärme steigt die Temperatur der Reaktionsmischung auf 130°C. Nach dem Abkühlen auf 90°C werden 225 g Wasser hinzugefügt und anschließend noch 7,5 g (30 mmol) Wolframsäure zugesetzt. Über einen Zeitraum von 6 h werden insgesamt 510,3 g (7,5 mol) 50 %iges Wasserstoffperoxid zudosiert. Anschließend wird 10 h bei dieser Temperatur nachgerührt. Nach dem Abkühlen erhält man eine klare gelbliche Polycarbonsäurelösung. Vor der Verwendung zur Ausrüstung der Cellulose-Fasern werden noch 53,6 g (0,67 mol) 50 %ige Natronlauge hinzugefügt.

### Beispiel A6:

In einem Rührgefäß werden 228,2 g (1,5 mol) Tetrahydrophthalsäureanhydrid und 135,1 g (1,5 mol) Milchsäure unter Rühren auf 100°C erhitzt. Durch die freiwerdende Reaktionswärme steigt die Temperatur der Reaktionsmischung auf 120°C. Nach dem Abkühlen auf 90°C werden 450 g Wasser hinzugefügt und anschließend noch 7,5 g (30 mmol) Wolframsäure zugesetzt. Über einen Zeitraum von 6 h werden insgesamt 510,3 g (7,5 mol) 50 %iges Wasserstoffperoxid zudosiert. Anschließend wird 10 h bei dieser Temperatur nachgerührt. Nach dem Abkühlen erhält man eine klare gelbliche Polycarbonsäurelösung. Vor der Verwendung zur Ausrüstung der Cellulose-Fasern werden noch 57,9 g (0,72 mol) 50 %ige Natronlauge hinzugefügt.

### Beispiel A7:

In einem Rührgefäß werden 228,2 g (1,5 mol) Tetrahydrophthalsäureanhydrid und 100,6 g (0,75 mol) 2,2-Bis-(hydroxymethyl)-propionsäure unter Rühren auf 135°C erhitzt. Durch die freiwerdende Reaktionswärme steigt die Temperatur der Reaktionsmischung auf 145°C. Nach dem Abkühlen auf 90°C werden 450 g Wasser hinzugefügt und anschließend noch 7,5 g (30 mmol) Wolframsäure zugesetzt. Über einen Zeitraum von 6h werden insgesamt 510,3 g (7,5 mol) 50%iges Wasserstoffperoxid zudosiert. Anschließend wird 10 h bei dieser Temperatur nachgerührt. Nach dem Abkühlen erhält man eine klare gelbliche Polycarbonsäurelösung. Vor der Verwendung zur Ausrüstung der Cellulose-Fasern werden noch 56,3 g (0,70 mol) 50 %ige Natronlauge hinzugefügt.

### Beispiel A8:

In einem Rührgefäß werden 228,2 g (1,5 mol) Tetrahydrophthalsäureanhydrid und 46,6 g (0,75 mol) Ethylenglykol unter Rühren auf 100°C erhitzt. Durch die freiwerdende Reaktionswärme steigt die Temperatur der Reaktionsmischung auf 130°C. Nach dem Abkühlen auf 90°C werden 450 g Wasser hinzugefügt und anschließend noch 9,9 g (30 mmol) Natriumwolframatdihydrat zugesetzt. Über einen Zeitraum von 6 h werden insgesamt 510,3 g (7,5 mol) 50 %iges Wasserstoffperoxid zudosiert. Anschließend wird 10 h bei dieser Temperatur nachgerührt. Nach dem Abkühlen erhält man eine klare gelbliche Polycarbonsäurelösung. Vor der Verwendung zur Ausrüstung der Cellulose-Fasern werden noch 53,0 g 50 %ige Natronlauge hinzugefügt.

### II Ausrüstung von Baumwolle

Zur Ausrüstung von 100 %iger Baumwolle mit einem Flächengewicht von 110 g/m² werden unterschiedliche Flotten mit in nachfolgenden Tabelle angegebener Zusammensetzung.hergestellt. Alle Inhaltsstoffe der Flotte sind dabei in g/l angegeben.

Zum Vergleich wird eine Flotte (V1) mit dem konventionellen, formaldehydhaltigen Vernetzungsmittel Dimethyloldihydroxylethylenharnstoff (DMDHEU) hergestellt.

Die Baumwolle wird zunächst in die Flotten getaucht und anschließend auf einem Foulard auf eine Flottenaufnahme von 70 bis 85 % abgequetscht. Das so präparierte Textil wird auf einem Spannrahmen 10 Minuten bei 120°C getrocknet und 5 Minuten bei 150°C kondensiert.

Bestimmt werden die Knittererholungswinkel nach DIN 53 890, die Maßänderung nach DIN 53 892 sowie das Selbstglättungsverhalten nach der Wäsche nach DIN 53 895. Zur Bestimmung der Maßänderung bzw. des Selbstglättungsverhalten werden die Proben nach DIN 53 920, Verfahren 3 A, gewaschen. Als Vergleich werden die entsprechenden Werte auch für die unbehandelte Baumwolle bestimmt.

**Tabelle 1:**

| | Beispiel | | | | | | | | | |
|---|---|---|---|---|---|---|---|---|---|---|
| | 1 | 2 | 3 | 4 | 5 | 6 | 7 | 8 | V1 | Rohware |
| Verwendetes Produkt | A1 | A2 | A3 | A4 | A5 | A6 | A7 | A8 | | |
| in g/l Flotte | 220 | 220 | 220 | 220 | 180 | 220 | 220 | 220 | | |
| NaH₂PO₂ | 30 | 30 | 30 | 30 | 30 | 30 | 30 | 30 | | |
| DMDHEU (ca.55%ig) | | | | | | | | | 110 | |
| MgCl₂ | | | | | | | | | 24 | |
| Essigsäure 60 % | | | | | | | | | 1 | |
| pH-Wert | 3 | 3 | 3 | 3 | 3 | 3 | 3 | 3 | 4 | |
| Knittererholungswinkel K+S nach DIN 53890 | 186° | 177° | 192° | 183° | 211° | 209° | 213° | 188° | 198° | 98° |
| Maßänderung K+S nach DIN 53892 in % | -0,7 | -0,7 | -0,6 | -0,7 | -0,8 | -0,8 | -0,6 | -0,7 | -0,8 | -2,4 |
| Selbstglättungsnote nach DIN 53895 | 6 | 6 | 6 | 6 | 6 | 6 | 6 | 6 | 6 | 1 |

## Patentansprüche

1. Verfahren zur Herstellung von Polycarbonsäuren durch
1) Umsetzung von Verbindungen der Formeln (I) oder (II) 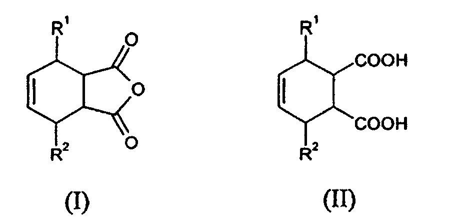 wobei
R¹ und R² gleich oder verschieden sind und H oder geradkettiges oder verzweigtes C₁-C₅-Alkyl bedeuten,
mit Trimethylolpropan, 2,2-Bis(hydroxymethyl)propionsäure oder mit Verbindungen der Formel (III)
R³XH (III)
wobei
X O, NH oder S und
R³ geradkettiges oder verzweigtes C₁-C₃₀-Alkyl,
geradkettiges oder verzweigtes C₂-C₃₀-Alkenyl,
C₅-C₁₂-Cycloalkyl,
-CHR⁴COOH,
wobei
R⁴ H, geradkettiges oder verzweigtes C₁-C₅-Alkyl, -CH₂OH, -CH(OH)COOH oder -CH2COOH bedeutet,
oder -(CH₂CR⁵R⁶Y)ₙR⁷,
wobei
Y für O oder NR⁸ steht,
R⁵, R⁶, R⁷ und R⁸ unabhängig voneinander H, geradkettiges oder verzweigtes C₁-C₄-Alkyl, -CH₂OH oder -CH₂CH₂OH bedeuten und
n eine ganze Zahl von 1 bis 20 darstellt
bedeuten und
2) anschließende Oxidation in Gegenwart von Wasserstoffperoxid oder einer Wasserstoffperoxid-freisetzenden Verbindung sowie eines Katalysators.

2. Verfahren nach Anspruch 1, **dadurch gekennzeichnet, dass** R¹ und R² gleich oder verschieden sind und in den Formeln (I) und (II) für Wassserstoff oder Methyl, bevorzugt beide für Wasserstoff stehen.

3. Verfahren nach Anspruch 1 oder 2, **dadurch gekennzeichnet, dass** Verbindungen der Formel (III) eingesetzt werden, bei denen
X O bedeutet und
R³ -CHR⁴COOH,
worin
R⁴ H, geradkettiges oder verzweigtes C₁-C₅-Alkyl, -CH₂OH, -CH(OH)COOH oder -CH₂COOH bedeutet,
oder -(CH₂CR⁵R⁶Y)ₙR⁷,
wobei
Y für O oder NR⁸ steht und
R⁵, R⁶, R⁷ und R⁸ unabhängig voneinander H, geradkettiges oder verzweigtes C₁-C₄-Alkyl, -CH₂OH oder - CH₂CH₂OH bedeuten und
n eine ganze Zahl von 1 bis 10, bevorzugt von 1 bis 5 darstellt.

4. Verfahren nach einem oder mehreren der Ansprüche 1 bis 3, **dadurch gekennzeichnet, dass** als Verbindungen der Formel (III) Ethylenglykol, Diethylenglykol, Triethylenglykol, Milchsäure oder Glycerin eingesetzt werden.

5. Verfahren nach einem oder mehreren der Ansprüche 1 bis 4, **dadurch gekennzeichnet**, dasss in Schritt 1) 1,2,3,6-Tetrahydrophthalsäureanhydrid mit einer Verbindung R³OH umgesetzt wird, wobei R³ für -(CH₂CR⁵R⁶Y)ₙR⁷ steht, Y für O oder NR⁸ steht und R⁵, R⁶, R⁷ und R⁸ unabhängig voneinander H, geradkettiges oder verzweigtes C₁-C₄-Alkyl, -CH₂OH oder -CH₂CH₂OH bedeuten und n eine ganze Zahl von 1 - 20, bevorzugt 1-10 und insbesondere 1 bis 5 darstellt.

6. Verfahren nach einem oder mehreren der Ansprüche 1 bis 5, **dadurch gekennzeichnet, dass** die Oxidation in Schritt 2) in Gegenwart eines Wolfram- oder Molybdän-haltigen Katalysators, bevorzugt Wolframoxid (WO₃), Wolfram säure (H₂WO₄ bzw. WO₃·H₂O), Isopolysäuren und Heteropolysäuren des Wolframs, Alkalimetall-, Erdalkalimetall oder Ammonium-Wolframaten, besonders bevorzugt Natriumwolframat (Na₂WO₄·2H₂O), Ammoniumparawolframat ((NH₄)₁₀W₁₂O₄₁·11H₂O)und Ammoniummetawolframat ((NH₄)₆H₂W₁₂O₄₀), Molybdän(VI)-oxid, Isopolysäuren und Heteropolysäuren des Molybdäns oder Alkalimetall-, Erdalkalimetall- oder Ammonium-Molybdaten durchgeführt wird.

7. Verfahren nach einem oder mehreren der Ansprüche 1 bis 6, **dadurch gekennzeichnet, dass** die Umsetzung der Verbindungen (I) oder (II) mit der Verbindung (III) in Schritt 1) bei einer Temperatur von 50 bis 250°C, bevorzugt 90 bis 150°C und einem Druck von 0-10 bar, bevorzugt 0,5 bis 2 bar erfolgt und die Oxidation in Schritt 2) unabhängig davon bei einer Temperatur von 50 bis 150°C, bevorzugt 70 bis 95°C und einem Druck von 0 bis 4 bar, bevorzugt 0,5 bis 2 bar durchgeführt wird.

8. Polycarbonsäuren erhältlich nach einem oder mehreren der Ansprüche 1 bis 7.

9. Verwendung der Polycarbonsäuren nach Anspruch 8 zur Ausrüstung von cellulosehaltigen Fasern oder daraus hergestellten Textil- oder Papiermaterialien.

10. Verfahren zur Ausrüstung von cellulosehaltigen Fasern oder daraus hergestellten Textil- oder Papiermaterialien, **dadurch gekennzeichnet, dass** die cellulosehaltigen Fasern oder daraus hergestellten Textil- oder Papiermaterialien mit einer wässrigen Flotte behandelt werden, die die Polycarbonsäure nach Anspruch 8 sowie gegebenenfalls Katalysatoren und Textilhilfsmittel, bevorzugt Weichmacher, Appreturmittel, Hydrophobiermittel, Oleophobiermittel, Flammschutzmittel oder pH-Regulatoren enthält.

11. Verfahren nach Anspruch 10, **dadurch gekennzeichnet, dass** als Katalysatoren Alkalimetallhypophosphite, Alkalimetallphosphite, bevorzugt Natrium-hypophosphit oder Natriumphosphit, Cyanamid oder eine Verbindung der Formel 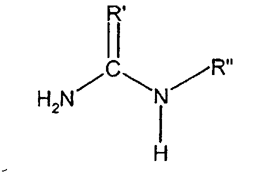 wobei
R' für NH, O oder S steht und
R" CN oder H bedeutet, verwendet werden.

12. Cellulosehaltige Fasern oder daraus hergestellten Textil- oder Papiermaterialien, die mit den Polycarbonsäuren nach Anspruch 8 behandelt wurden.

## Claims

1. Process for preparing polycarboxylic acids by
1) reacting compounds of the formulae (I) or (II) 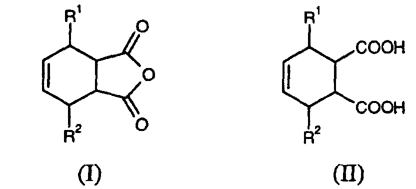 where
R1 and R2 are identical or different and each is H or straight-chain or branched C1-C5-alkyl,
with trimethylolpropane, 2,2-bis(hydroxymethyl)propionic acid or with compounds of the formula (III)
R3XH (III)
where
X is O, NH or S and
R3 is straight-chain or branched C1-C30-alkyl,
straight-chain or branched C2-C30-alkenyl,
C5-C12-cycloalkyl,
-CHR4COOH,
where
R4 is H, straight-chain or branched C1-C5-alkyl -CH2OH, -CH(OH)COOH or -CH2COOH,
or -(CH2CR5R6Y)nR7,
where
Y is O or NR8,
R5, R6, R7 and R8 are independently H, straight-chain or branched C1-C4-alkyl, -CH2OH or -CH2CH2OH and
n is an integer from 1 to 20
and
2) subsequent oxidation in the presence of hydrogen peroxide or of a hydrogen peroxide releaser and of a catalyst.

2. Process according to Claim 1, **characterized in that** R1 and R2 are identical or different and are each hydrogen or methyl, preferably both hydrogen, in the formulae (I) and (II).

3. Process according to Claim 1 or 2, **characterized by** use of compounds of the formula (III) where
X is O and
R3 is -CHR4COOH,
where
R4 is H, straight-chain or branched C1-C5-alkyl, -CH2OH,
-CH(OH)COOH or -CH2COOH,
or -(CH2CR5R6Y)nR7,
where
Y is O or NR8 and
R5, R6, R7 and R8 are independently H, straight-chain or branched C1-C4-alkyl, -CH2OH or - CH2CH2OH and
n is an integer from 1 to 10, preferably from 1 to 5.

4. Process according to one or more of Claims 1 to 3, **characterized in that** compounds of the formula (III) are ethylene glycol, diethylene glycol, triethylene glycol, lactic acid or glycerol.

5. Process according to one or more of Claims 1 to 4, **characterized in that** step 1) is effected by reacting 1,2,3,6-tetrahydrophthalic anhydride with an R30H compound, where R3 is -(CH2CR5R6Y)nR7, Y is O or NR8 and R5, R6, R7 and R8 are independently H, straight-chain or branched C1-C4-alkyl, -CH20H or -CH2CH2OH and n is an integer from 1-20, preferably 1-10, especially 1 to 5.

6. Process according to one or more of Claims 1 to 5, **characterized in that** the oxidation in step 2) is carried out in the presence of a tungsten or molybdenum catalyst, preferably tungsten oxide (W03), tungstic acid (H2WO4 or W03 . H2O), isopolyacids and heteropolyacids of tungsten, alkali metal, alkaline earth metal or ammonium tungstates, particularly preferably sodium tungstate (Na2WO4.2H2O), ammonium paratungstate ((NH4)10W12O41.11H2O) and ammonium metatungstate ((NH4)6H2W12O40), molybdenum(VI) oxide, isopolyacids and heteropolyacids of molybdenum or alkali metal, alkaline earth metal or ammonium molybdates.

7. Process according to one or more of Claims 1 to 6, **characterized in that** the reaction of compounds (I) or (II) with (III) in step 1) is carried out at a temperature of 50 to 250°C, preferably 90 to 150°C, and a pressure of 0-10 bar, preferably 0.5 to 2 bar, and the oxidation in step 2) is independently carried out at a temperature of 50 to 150°C, preferably 70 to 95°C, and a pressure of 0 to 4 bar, preferably 0.5 to 2 bar.

8. Polycarboxylic acids obtainable according to one or more of Claims 1 to 7.

9. Use of the polycarboxylic acids according to Claim 8 for finishing cellulosic fibres or textile or paper materials produced therefrom.

10. Process for finishing cellulosic fibres or textile or paper materials produced therefrom, **characterized in that** the cellulosic fibres or textile or paper materials produced therefrom are treated with an aqueous liquor containing the polycarboxylic acid according to Claim 8 with or without catalysts and textile auxiliaries, preferably softeners, hand modifiers, hydrophobicizers, oleophobicizers, flame retardants or pH regulators.

11. Process according to Claim 10, **characterized in that** catalysts used are alkali metal hypophosphites, alkali metal phosphites, preferably sodium hypophosphite or sodium phosphite, cyanamide or a compound of the formula 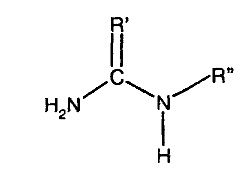 where
R' is NH, O or S and
R" is CN or H.

12. Cellulosic fibres or textile or paper materials treated with the polycarboxylic acids according to Claim 8.

## Revendications

1. Procédé de préparation d'acides polycarboxyliques par
(1) réaction de composés des formules (I) ou (II) 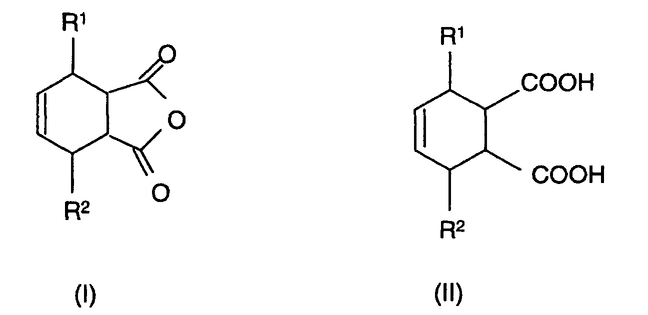 où
R¹ et R² sont identiques ou différents et représentent H ou alkyle en C₁-C₅ linéaire ou ramifié,
avec le triméthylolpropane, l'acide 2,2-bis(hydroxyméthyl)propionique ou avec des composés de formule (III)
R³XH (III)
où
X représente O, NH ou S et
R³ représente alkyle en C₁-C₃₀ linéaire ou ramifié,
alcényle en C₂-C₃₀ linéaire ou ramifié,
cycloalkyle en C₅-C₁₂,
-CHR⁴COOH,
où
R⁴ représente H, alkyle en C₁-C₅ linéaire ou ramifié, -CH₂OH, -CH(OH)COOH ou -CH₂COOH,
ou -(CH₂CR⁵R⁶Y)ₙR⁷,
où
Y représente O ou NR⁸,
R⁵, R⁶, R⁷ et R⁸ représentent indépendamment les uns des autres H, alkyle en C₁-C₄ linéaire ou ramifié, -CH₂OH ou -CH₂CH₂OH et
n représente un nombre entier de 1 à 20
puis
2) oxydation en présence de peroxyde d'hydrogène ou d'un composé libérant du peroxyde d'hydrogène ainsi que d'un catalyseur.

2. Procédé selon la revendication 1, **caractérisé en ce que** R¹ et R² sont identiques ou différents et représentent dans les formules (I) et (II) l'hydrogène ou méthyle, de préférence l'un et l'autre l'hydrogène.

3. Procédé selon la revendication 1 ou 2, **caractérisé en ce que** l'on utilise des composés de formule (III) où
X représente O et
R³ représente -CHR⁴COOH,
où
R⁴ représente H, alkyle en C₁-C₅ linéaire ou ramifié, -CH₂OH, -CH(OH)COOH ou -CH₂COOH,
ou -(CH₂CR⁵R⁶Y)ₙR⁷,
où
Y représente O ou NR⁸ et
R⁵, R⁶, R⁷ et R⁸ représentent indépendamment les uns des autres H, alkyle en C₁-C₄ linéaire ou ramifié, -CH₂OH ou -CH₂CH₂OH et
n représente un nombre entier de 1 à 10, de préférence de 1 à 5.

4. Procédé selon une ou plusieurs des revendications 1 à 3, **caractérisé en ce que** l'on utilise comme composés de formule (III) l'éthylèneglycol, le diéthylèneglycol, le triéthylèneglycol, l'acide lactique ou la glycérine.

5. Procédé selon une ou plusieurs des revendications 1 à 4, **caractérisé en ce que**, dans l'étape 1), l'anhydride 1,2,3,6-tétrahydrophthalique est mis à réagir avec un composé R³OH où R³ représente -(CH₂CR⁵R⁶Y)ₙR⁷, Y représente O ou NR⁸ et R⁵, R⁶, R⁷ et R⁸ représentent indépendamment les uns des autres H, alkyle en C₁-C₄ linéaire ou ramifié, -CH₂OH ou -CH₂CH₂OH et n représente un nombre entier de 1-20, de préférence 1-10 et en particulier 1 à 5.

6. Procédé selon une ou plusieurs des revendications 1 à 5, **caractérisé en ce que** l'oxydation dans l'étape 2) est conduite en présence d'un catalyseur contenant du tungstène ou du molybdène, de préférence de l'oxyde de tungstène (WO₃), de l'acide tungstique (H₂WO₄ ou WO₃,H₂O), des isopolyacides et des hétéropolyacides du tungstène, des tungstates de métaux alcalins, de métaux alcalino-terreux ou d'ammonium, de manière particulièrement préférée du tungstate de sodium (Na₂WO₄,2H₂O), du paratungstate d'ammonium ((NH₄)₁₀W₁₂O₄₁,11H₂O) et du métatungstate d'ammonium ((NH₄)₆H₂W₁₂O₄₀), de l'oxyde de molybdène (VI), des isopolyacides et des hétéropolyacides du molybdène ou des molybdates de métaux alcalins, de métaux alcalino-terreux ou d'ammonium.

7. Procédé selon une ou plusieurs des revendications 1 à 6, **caractérisé en ce que** la réaction des composés (I) ou (II) avec le composé (III) dans l'étape 1) est conduite à une température de 50 à 250°C, de préférence de 90 à 150°C et à une pression de 0-10 bar, de préférence de 0,5 à 2 bar et l'oxydation dans l'étape 2) est conduite indépendamment de celle-ci à une température de 50 à 150°C, de préférence de 70 à 95°C et à une pression de 0 à 4 bar, de préférence de 0,5 à 2 bar.

8. Acides polycarboxyliques pouvant être obtenus selon l'une ou plusieurs des revendications 1 à 7.

9. Utilisation des acides polycarboxyliques selon la revendication 8 pour l'apprêt de fibres cellulosiques ou de matériaux textiles ou de type papier produits à partir de celles-ci.

10. Procédé pour apprêter des fibres cellulosiques ou des produits textiles ou de type papier produits à partir de celles-ci, **caractérisé en ce que** les fibres cellulosiques ou les produits textiles ou de type papier produits à partir de celles-ci sont traités avec un bain aqueux qui contient l'acide polycarboxylique selon la revendication 8 ainsi éventuellement que des catalyseurs et des adjuvants textiles, de préférence des plastifiants, des apprêts, des agents hydrophobants, des agents oléophobants, des ignifugeants ou des régulateurs de pH.

11. Procédé selon la revendication 10, **caractérisé en ce que** l'on utilise comme catalyseurs des hypophosphites de métaux alcalins, des phosphites de métaux alcalins, de préférence l'hypophosphite de sodium
ou le phosphite de sodium, le cyanamide ou un composé de formule 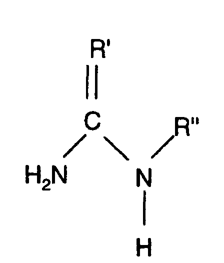 où
R' représente NH, O ou S et
R" représente CN ou H.

12. Fibres cellulosiques, ou matériaux textiles ou de type papier produits à partir de celles-ci, qui ont été traitées avec les acides polycarboxyliques selon la revendication 8.
